(19) Europäisches Patentamt
European Patent Office
Office européen des brevets

(11) Veröffentlichungsnummer: **0 004 591**
**B1**

(12) # EUROPÄISCHE PATENTSCHRIFT

(45) Veröffentlichungstag der Patentschrift: **31.03.82**

(51) Int. Cl.³: **B 01 J 23/22**, C 07 C 50/18

(21) Anmeldenummer: **79100838.6**

(22) Anmeldetag: **20.03.79**

(54) **Schalenkatalysatoren, Verfahren zu ihrer Herstellung und ihre Verwendung für die Bereitung von Anthrachinonen.**

(30) Priorität: **03.04.78 DE 2814262**

(43) Veröffentlichungstag der Anmeldung: **17.10.79 Patentblatt 79/21**

(45) Bekanntmachung des Hinweises auf die Patenterteilung: **31.03.82 Patentblatt 82/13**

(84) Benannte Vertragsstaaten: **BE DE FR GB IT NL**

(56) Entgegenhaltungen:
**DE-A-2 547 624**
**DE-A-2 547 655**
**DE-B-2 160 781**

(73) Patentinhaber: **BASF Aktiengesellschaft, Carl-Bosch-Strasse 38, D-6700 Ludwigshafen (DE)**

(72) Erfinder: **Engelbach, Heinz, Dr., Kropsburgstrasse 24, D-6703 Limburgerhof (DE)**
Erfinder: **Hoffmann, Harry, Bauernwiesenstrasse 82, D-6700 Ludwigshafen (DE)**
Erfinder: **Palm, Peter, Hauptstrasse 46, D-6711 Gerolsheim (DE)**
Erfinder: **Sommer, Karl, Dr., Bozener Strasse 19, D-6700 Ludwigshafen (DE)**
Erfinder: **Sprague, Michael Jolyon, Dr., Lachnerstrasse 3, D-6800 Mannheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Schalenkatalysatoren, Verfahren zu ihrer Herstellung und ihre Verwendung für die Bereitung von Anthrachinonen

Die Erfindung betrifft neue Schalenkatalysatoren und Verfahren zu ihrer Herstellung durch Zerstäubung von Gemischen aus Wasser und Vanadium-V-Verbindungen und gegebenenfalls weiteren Metallverbindungen und Auftrag auf sich in Eigenbewegung befindende Trägerteilchen, Trocknung sowie wiederholte kurzfristige Behandlung der sich in Eigenbewegung befindlichen Katalysatoren mit Flammen oder Plasma und rasche Abkühlung unter bestimmten Bedingungen der Auftragsgeschwindigkeit.

Es ist bekannt, für katalystische Reaktionen Schalenkatalysatoren zu verwenden, die aus Trägern, z. B. in Form von Kugeln, Tabletten oder Strangpreßlingen bestehen, deren Oberfläche schalenförmig mit einer katalytisch aktiven Masse belegt ist. Als Träger werden nicht poröse oder wenig poröse inerte Materialien verwendet. Die Beschichtung dieser Träger mit der aktiven Masse erfolgt in der Weise, daß man die katalytisch aktiven Substanzen mit Wasser oder einem organischen Lösungsmittel zu einem Brei anrührt und den beispielsweise in einer Dragiertrommel in Bewegung gehaltenen Träger durch allmähliche Zugabe des Breies beschichtet. Hierbei werden Schalenkatalysatoren erhalten, deren Schalendicke zwischen etwa 0,02 bis 4 Millimeter liegt. Anschließend müssen die mit den aktiven Komponenten beschichteten Katalysatoren längere Zeit auf höhere Temperaturen erhitzt werden.

Aus der DE-A-2 045 430 ist bekannt, daß man Schalenkatalysatoren, bestehend aus einem Träger und einer darauf aufgebrachten Schicht aus katalytisch aktiver Masse dadurch herstellen kann, daß man die katalytisch aktive Masse mit Hilfe des Plasmaspritz- oder Flammspritzverfahrens auf den Träger aufbringt. Wie die Patentschrift angibt, sind die Trägerteilchen vorteilhaft kugelförmig, um den Druckverlust im Reaktor niedrig zu halten. Sie können z. B. in einer Pelletisiertrommel hergestellt sein. Voraussetzung für die Anwendung des Verfahrens ist die Schmelzbarkeit mindestens einer Hauptkomponente bei der Arbeitstemperatur des Flammspritzbrenners, der mit Acetylen/Sauerstoff betrieben wird, oder des Plasmabrenners. Das Verfahren wird in der Weise durchgeführt, daß man die aktiven Komponenten oder die Substanzen, die sich bei der Arbeitstemperatur der Brenner in die gewünschten oxidischen und katalytisch aktiven Verbindungen zersetzen lassen, in die Flamme bzw. den Plasmastrahl in bekannter Weise einbringt. Die Flamme bzw. der Plasmastrahl wird auf die zu beschichtenden Teilchen gerichtet, die vorzugsweise z. B. in einer rotierenden Trommel in Bewegung gehalten werden. Die so hergestellten Katalysatoren werden z. B. für die Oxidation von Indanen verwendet, wobei als katalytisch aktive Schichten Vanadinpentoxidschichten oder Schichten aus Molybdänoxid und/oder Wolframoxid auf Trägerteilchen aufgebracht werden.

In den Beispielen wird gezeigt, daß im Vergleich zu üblichen Verfahren die katalytische Wirkung der Schalenkatalysatoren erheblich gerade dadurch gesteigert werden kann, daß man die katalytische Masse, z. B. Vanadinpentoxidpulver, als geschmolzene Masse über Flammspritz- oder Plasmabrenner auf den Träger aufspritzt und gegebenenfalls anschließend den Katalysator noch eine Stunde bei 650 bis 700° C kalziniert. Denn das Vergleichsbeispiel zeigt einen Auftrag der katalytischen Masse mittels Zerstäuber auf die Trägerkugeln bei einer Temperatur von 200° C; die beschichteten Kugeln werden dann jeweils eine Stunde lang auf 200, 300, 500 und 700° C in Luftatmosphäre erhitzt. Die auf diese bekannte Weise des Auftrags mittels Zerstäubung erhaltenen Katalysatoren liefern im Vergleich zu den Ausbeuten des in der DE-A-2 045 430 beschriebenen Verfahrens (45 Prozent) nur eine Ausbeute von 13,8 Prozent.

Obwohl das vorgenannte Verfahren im Hinblick auf ältere Verfahren bessere Ergebnisse, insbesondere in der Ausbeute an Endstoff, liefert, ist es doch nicht völlig befriedigend. Beim Plasma- und beim Flammspritzverfahren muß die aktive Masse dem Brenner mittels pneumatischer Förderung zugeführt werden. Diese Art der Zuführung erfordert ein gut rieselfähiges Material und läßt sich nur dann völlig störungsfrei durchführen, wenn der Kornbereich der aktiven Masse etwa zwischen 50 und 200 µm liegt und das Einzelkorn möglichst kugelförmig ist. Die Herstellung einer solchen Kornform und Kornverteilung ist im großtechnischen Maßstab mit Schwierigkeiten verbunden. Sie gelingt am besten durch Schmelzen und anschließendes Versprühen der aktiven Masse mit einem Gasstrom, wobei das Sprühgut mit einer Körnung $<50$ µ und $>200$ µ zurückgeführt wird. Nachteilig ist bei dieser Herstellung der optimalen Kornverteilung der hohe Energieverbrauch sowie die wegen der Giftigkeit von z. B. Vanadin erforderlich werdende kostspielige Gasreinigung mittels Wäsche und Elektroabscheider. Eine ähnlich aufwendige Gasreinigung ist aber auch für den Flammspritzvorgang selber erforderlich, da nur 50 bis 70 Prozent der dem Brenner zugeführten aktiven Masse auf den Trägerkugeln aufgeschmolzen wird; der Rest muß aus dem Abgas zurückgewonnen werden.

Es ist aus den DE-A-1 934 063, 2 028 424, 2 050 797, 2 060 215, 2 122 664, 2 135 421, 2 160 781, 2 314 695 und der deutschen Offenlegungsschrift 2 224 016 bekannt, daß man vorteilhaft Indane in Gegenwart von Vanadinpentoxid und/oder Vandaten von weiteren Elementen oxidiert. Es wird beschrieben, daß die Katalysatoren durch Fällung oder durch Eindampfen einer Lösung von Ammoniumvanadat mit den entsprechenden Metallsalzen, z. B. Lösungen von Antimontartrat, Ferrinitrat und Caesiumnitrat oder von Rubidiumnitrat und Titan-(III)-oxalat, Filtration und Trocknen des Metallvanadatniederschlags hergestellt werden können. Durch Zugabe des Trägermaterials vor oder während der Fällung wird das

Metallvanadat gleichzeitig auf dem Träger fein verteilt. Ebenfalls kann die Lösung oder Suspension des Vanadats durch Tränken oder Sprühen auf den Träger aufgebracht werden. Auch ist es möglich, das trockene und feuchte Vanadat mit dem Träger zu vermischen, gegebenenfalls das Gemisch zu zerkleinern und dann, z. B. mit Hilfe einer Strangpresse, in entsprechende Formlinge überzuführen. Nach dem Trocknen kann der Katalysator noch vorteilhaft calciniert werden, z. B. bei einer Temperatur zwischen 300 und 700°C. Zur Herstellung von Vanadinpentoxidkatalysatoren kann man z. B. Vanadinpentoxid in wäßriger Oxalsäure oder Salzsäure lösen und die Lösung auf einen geeigneten Träger, z. B. Titandioxid oder Steatit, auftragen, den Träger trocknen und gegebenenfalls calcinieren. Entsprechend kann man auch Lösungen von Ammoniumvanadat in Wasser verwenden. Jedoch wird ausdrücklich darauf hingewiesen, z. B. schon in der deutschen Patentschrift 2 028 424, daß sich als besonders vorteilhafte Ausführungsform die Herstellung der Katalysatoren auf kugelförmigen Trägern nach einem Flammspritz- oder Plasmaspritzverfahren, z. B. nach dem in der deutschen Patentschrift 2 025 430 beschriebenen Verfahren, erweist. Die vorgenannten Verbindungen anderer Elemente können mit dem mittels Flammspritzen aufzubringenden Vanadinpentoxid oder einer Verbindung, die beim Erhitzen in Vanadinpentoxid übergeht, wie z. B. Vanadinsäure, mechanisch vermischt werden. Es wird jedoch ebenfalls als vorteilhaft beschrieben, zunächst eine homogene Lösung herzustellen, die die aufzutragenden Metalle enthält. Aus dieser Lösung können die aufzutragenden Verbindungen, z. B. durch Eindampfen, gewonnen werden. Wie die Beispiele, z. B. die in der deutschen Patentschrift 2 028 424 beschriebenen, zeigen, wird in der Regel eine katalytische Masse aus Verbindungen vorgenannter Elemente hergestellt und diese dann durch Flammspritzen in der Arbeitsweise der deutschen Patentschrift 2 025 430 auf den Träger aufgebracht. Die deutschen Patentschriften 2 050 797, 2 122 664, 2 135 421 und die deutsche Offenlegungsschrift 2 224 016 lehren daneben als vorteilhafte Arbeitsweise, die aktive Masse mittels Flammspritz- oder Plasmaspritzverfahren auf den Träger aufzubringen und den Katalysator anschließend bei 450 bis 650°C, vorzugsweise von 500 bis 600°C, während 1 bis 24 Stunden zu calcinieren.

Die DE-A-2 547 624 beschreibt die Herstellung von Trägerkatalysatoren, die Rubidium, Antimon, Vanadiumpentoxid und Titandioxid enthalten. Die entsprechenden Metallverbindungen von breiförmiger Konsistenz werden z. B. in einer Dragiertrommel auf den auf 100 bis 450°C vorerhitzten Träger aufgesprüht. Gegebenenfalls wird noch bei 200 bis 500°C nacherhitzt. Die DE-A-2 547 635 beschreibt Katalysatoren, die Vanadinpentoxid und Titandioxid und/oder Zirkondioxid enthalten. Die Metallverbindungen können als Suspension auf den auf 160 bis 500°C erhitzten Träger aufgesprüht werden, gegebenenfalls wird in weiterer Operation der Katalysator bei 300 bis 700°C calciniert.

Es wurde nun gefunden, daß man Schalenkatalysatoren, bestehend aus einem nicht oder wenig porösen Träger und einer darauf aufgebrachten Schicht aus katalytisch aktiver, Vanadium-V-Verbindungen enthaltender Masse, wobei man Vanadium-V-Verbindungen und gewünschtenfalls andere Metallverbindungen im Gemisch mit Wasser zerstäubt und das so zerstäubte Gut auf sich in ständiger Eigenbewegung befindende Trägerteilchen aufbringt, vorteilhaft erhält, wenn man das Gut bei einer Temperatur von 20 bis 90°C und mit einer Auftragsgeschwindigkeit von 0,001 bis 0,02 Gewichtsteilen Feststoff je Volumenteil Trägerkugeln mal Minute als Schale aufträgt, dann die so erhaltenen Schalenkatalysatoren trocknet und mit einer Gesamtbehandlungszeit der Schalenkatalysatoren von 2 bis 60 Minuten mittels einer Flammerhitzung oder Plasmaerhitzung der sich in ständiger Eigenbewegung befindenden Schalenkatalysatoren wiederholt alle Teile der Katalysatoroberfläche kurzfristig auf eine Temperatur oberhalb 700°C erhitzt und während 0,1 bis 1 Sekunde auf eine Temperatur unterhalb 650°C abkühlt.

Weiterhin wurde gefunden, daß man die Schalenkatalysatoren vorteilhaft zur Herstellung von Anthrachionen durch Oxidation von Indanen der Formel

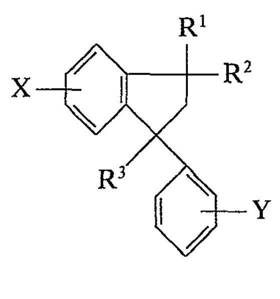

I

in der $R^1$, $R^2$ und $R^3$ gleich oder verschieden sein können und jeweils einen Alkylrest bedeuten, $R^1$ und/oder $R^3$ darüber hinaus auch jeweils ein Wasserstoffatom bezeichnen können, die Reste X und Y gleich oder verschieden sein können und jeweils ein Halogenatom oder ein Wasserstoffatom bedeuten, mit Sauerstoff in der Gasphase in Gegenwart von Vanadium-V-Verbindungen und gewünschtenfalls von einer oder mehreren Verbindungen weiterer Metalle, verwendet.

Die Erfindung beruht auf der Beobachtung, daß optimale Vanadiumschalenkatalysatoren, die besonders für die Oxidation von Indanen zu Anthrachionen geeignet sind, nicht allein auf ihrer Zusammensetzung bezüglich der Zusatzelemente und ihrer Gewichtsrelationen zum Anteil an Vanadin,

sondern wesentlich auch auf einer spezifischen Kombination von Merkmalen der Katalysatorstruktur beruhen, die von den folgenden Faktoren besonders bestimmt werden: Auftrag von Suspensionen bei bestimmter Auftragsgeschwindigkeit und Auftragstemperatur, rollender Eigenbewegung des Trägers beim Auftrag; Behandlung des Katalysators innerhalb eines ausgewählten und kurzen Zeitraumes bei sehr hoher Temperatur, die das Vanadinpentoxid und gegebenenfalls die zusätzlichen Metalloxide und -vanadate innerhalb der katalytisch aktiven Masse ganz oder teilweise während dieses Zeitraumes zum Schmelzen bringt; Durchführung dieser Hitzebehandlung in Form einer Flammspritz- oder Plasmaspritzbehandlung; rasche Abkühlung unter bestimmten Bedingungen der Temperatur; mehrfache Wiederholung von Erhitzung und Abkühlung der Katalysatoroberfläche. Im Hinblick auf die bekannten Verfahren liefert das Verfahren nach der Erfindung auf einfacherem und wirtschaftlicherem Wege Vanadinpentoxid enthaltende Schalenkatalysatoren. Verwendet man die Katalysatoren zur Oxidation von Indanen, so erhält man die entsprechenden Anthrachinone in guter Ausbeute und Reinheit. Die Förderung der aktiven Masse über das Flammspritzgerät wird vermieden, da dieses nur zur kurzfristigen Erhitzung der Katalysatorschale verwendet wird; entsprechend spielen Rieselfähigkeit, Korngröße und Struktur der aktiven Masse vergleichsweise keine oder nur eine weit geringere Rolle. Man kann z. B. Korngrößen der aktiven Masse von 50 Mikrometern bis weniger als 1 Mikrometer verwenden, die für das Flammspritzverfahren unzweckmäßig sind. Eine besondere Zubereitung der aktiven Teilchen, z. B. durch Schmelzen und Versprühen der aktiven Masse im Gasstrom wird vermieden, Energie wird eingespart, zusätzliche Reinigungsoperationen des Abgases sind nicht notwendig. Da die aktive Masse nicht über das Flammspritzgerät zerstäubt wird, gelangen entsprechende Anteile nicht in das Abgas und brauchen deshalb nicht abgetrennt und zurückgeführt werden. Die Katalysatoren sind daher gerade im großtechnischen Maßstab einfacher, störungssicherer, wirtschaftlicher und umweltfreundlicher herzustellen. Im Vergleich zu den gemäß DE-A-2 547 624 und DE-A-2 547 655 erhältlichen Ausbeuten ist die erfindungsgemäße Ausbeute wesentlich höher. Alle diese vorteilhaften erfindungsgemäßen Ergebnisse sind im Hinblick auf den Stand der Technik überraschend.

Als aktive Masse verwendet man eine oder mehrere Vanadium-V-Verbindungen, vorzugsweise Vanadinpentoxid und/oder Vanadate. Die Vanadium-V-Verbindung kann im Katalysator gegebenenfalls auch im Gemisch mit der entsprechenden Vanadium-IV-Verbindung vorliegen. Die Vanadate können Mono- oder Poly-vanadate, insbesondere Ortho-, Pyro-, Metavanadate, sein. Unter den Vanadaten sind die Vanadate von Elementen der Gruppen Ia, IIb, IIIa, IIIb, IVa, IVb, Va, Vb, VIa, VIb, VIIb und VIIIb des Periodischen Systems bevorzugt, insbesondere Ammonium-, Kalium-, Thallium-, Bor-, Tellur-, Cadmium-, Germanium-, Niob-, Blei-, Rubidium-, Chrom-, Wolfram-, Uran-, Arsen-, Indium-, Caesium-, Zink-, Molybdän-, Eisen-, Titan-, Zinn-, Antimon- und Mangan-vanadat. Ebenfalls kommen Vanadium-V-Verbindungen im Gemisch mit Oxiden von Elementen der Gruppen Ia, IIb, IIIa, IIIb, IVa, IVb, Va, Vb, VIa, VIb, VIIb und/oder VIIIb des Periodischen Systems (Periodisches System gemäß d'Ans-Lax, Taschenbuch für Chemiker und Physiker, 3. Auflage (1967), Band I, Seite 63) für die Umsetzung als aktive Masse in Betracht, vorteilhaft mit den Oxiden des Kaliums, Thalliums, Bors, Tellurs, Cadmiums, Germaniums, Niobs, Bleis, Rubidiums, Chrom, Wolframs, Urans, Arsens, Indiums, Caesiums, Zinks, Molybdäns, Eisens, Titans, Zinns, Antimons und Mangans.

Auch Gemische von Vanadium-V-Verbindungen und zusätzlichen Verbindungen von Elementen, die unter den Reaktionsbedingungen Vanadate und/oder Oxide bilden, sind geeignet, z. B. eine oder mehrere Vanadin-V-Verbindungen und eine oder mehrere Verbindungen von Elementen der Gruppen Ia, IIb, IIIa, IIIb, IVa, IVb, Va, Vb, VIa, VIb, VIIb und/oder VIIIb des Periodischen Systems, insbesondere Verbindungen des Kaliums, Thalliums, Bors, Tellurs, Cadmiums, Germaniums, Niobs, Bleis, Rubidiums, Chroms, Wolframs, Urans, Arsens, Indiums, Caesiums, Zinks, Molybdäns, Eisens, Titans, Zinns, Antimons und Mangans.

Unabhängig von der Zusammensetzung der Verbindungen und der Wertigkeit der entsprechenden Metalle in den Verbindungen, die die aktive Masse enthält, ist im Katalysator zweckmäßig das Atomverhältnis von Vanadin zu den Zusatzelementen der Gruppe Ia von 2000 bis 0,5 Vanadium zu 1 Zusatzelement, in der Gruppe IIIa von 4000 bis 1 Vanadium zu 1 Zusatzelement, in der Gruppe IVa von 2000 bis 0,1 Vanadium zu 1 Zusatzelement, in der Gruppe Va von 2000 bis 1 Vanadium zu 1 Zusatzelement, in der Gruppe VIa von 2000 bis 1 Vanadium zu 1 Zusatzelement, in der Gruppe VIIb von 2000 bis 3 Vanadium zu 1 Zusatzelement, in der Gruppe VIIIb von 100 000 bis 0,5 Vanadium zu 1 Zusatzelement, in der Gruppe IIb von 2000 bis 3 Vanadium zu 1 Zusatzelement, in der Gruppe IIIb von 2000 bis 1 Vanadium zu 1 Zusatzelement, in der Gruppe IVb von 2000 bis 0,1 Vanadium zu 1 Zusatzelement, in der Gruppe Vb von 4000 bis 5 Vanadium zu 1 Zusatzelement und in der Gruppe VIb von 2000 bis 4 Vanadium zu 1 Zusatzelement.

Die Verbindungen der Zusatzelemente können beliebig gewählt werden, im allgemeinen kommen die Oxide, Säuren, Basen, Salze, z. B. Carbonate, Bicarbonate, Oxalate, Chloride oder Nitrate, Ammoniumsalze von Metallsäuren, und solche Verbindungen der Zusatzelemente in Frage, die sich während der Katalysatorherstellung zu den entsprechenden Oxiden und/oder Vanadaten umsetzen. Besonders vorteilhafte Zusatzelemente sind Antimon und Thallium. Bevorzugt ist ein Atomverhältnis von 30 000 bis 10, insbesondere 6000 bis 25 Vanadium zu 1 Thallium und von 8000 bis 3, insbesondere 1600 bis 8 Vanadium zu 1 Antimon. Als Zusatzverbindungen wählt man zweckmäßig solche mit einem

Fp unterhalb 1200°C. Solche Verbindungen sind insbesondere in den Fällen, in denen das Zusatzelement in größerer Menge im Katalysator vorliegt, bevorzugt. Gegebenenfalls ist bei höherschmelzenden Verbindungen ein Plasmabrenner zu verwenden. In diesem Falle vermeidet man zweckmäßig eine teilweise Reduktion des Vanadium-V zu Vanadium-IV, um die Bildung von höherschmelzendem Vandium-IV zu verhindern.

Als Zusatzverbindungen sind beispielsweise geeignet: Kaliumchlorid, Kaliumcarbonat, Bortrioxid, Thalliumnitrat, Borsäure, Kaliumhydroxid, Antimontrioxid, Antimontetroxid, -pentoxid, Antimontrioxid-hydrate, Antimonpentoxid-hydrate, Kaliumnitrat, Kaliumbicarbonat, Kaliumoxalat, -formiat, Germaniumoxid, Ammoniummolybdat, Niobnitrat, Wolframnitrat, Uranacetat, Indiumnitrat, Urandioxid, Thalliumacetat, Thalliumcarbonat, Thalliumvanadat, Ammoniumborat, Caesiumcarbonat, Caesiumnitrat, Caesiumbicarbonat, Caesiumoxalat, Caesiumformiat, Caesiumacetat, Caesiumhydrogentartrat, Zinksulfat, Cadmiumcarbonat, Zinkoxid, Zinknitrat, Zinkhydroxid, Cadmiumoxid, Cadmiumnitrat, Zinkoxalat, Zinkformiat, Cadmiumacetat, Zinkcarbonat, Cadmiumsulfat, Cadmiumhydroxid, Zinkacetat, Sauerstoffsäuren des Tellurs, Ammoniumtellurat, Tellurdioxid, Kaliumtellurit, Tellurtrioxid, Caesiumtellurat, Kaliumtellurat, Thalliumtellurat.

Neben einer oder mehreren Vanadium-V-Verbindungen können eine oder mehrere Zusatzverbindungen, z. B. Oxide, anderer Elemente, vorzugsweise die vorgenannten Verbindungen, z. B. Oxide, der vorgenannten Elemente, im Katalysator anwesend sein. Vorteilhaft sind neben der Kombination von 2 Verbindungen auch Kombinationen von Verbindungen des Vanadiums V mit Verbindungen von 2, 3, 4 oder 5 Elementen. Besonders bevorzugt ist die Kombination der Verbindungen von Vanadium, Antimon und Thallium. Ebenfalls kann man Salze der genannten Metalle mit Sauerstoffsäuren, die aus entsprechenden vorgenannten Metallen wie Wolfram gebildet sind, verwenden; beispielsweise kommen Eisenwolframat, Caesiumtellurat, Rubidiumwolframat in Betracht. Gegebenenfalls kann Vanadium nur in Gestalt von Vanadaten der bevorzugten Metalle vorliegen.

Als Trägerteilchen verwendet man nichtporöse oder wenig poröse Materialien, z. B. Kieselsäureverbindungen wie Silikate, z. B. Natriumaluminiumsilikat, Magnesiumsilikat, Calciumaluminiumsulikat, Bleicherden, Fullererde, Tone, Kaolin, Meerschaum, Allophane, Zeolithe, Montmorrilonit, Bimsstein, Floridaerde, Quarz, Asbest, Mullit, Steatit, Bentonit; gefällte Kieselsäure, Kieselgel, Kieselgur, Siliciumcarbid; $\alpha$- und $\gamma$-Aluminiumoxide und -hydroxide; z. B. Korund, $\gamma$-Tonerde, Hydrargillit, Böhmit, Bauxit; Aluminiumsilikate, z. B. Andalusit; Titandioxid, Zirkondioxid, Zinndioxid, Thoriumdioxid, Magnesit, Zinkoxid. Bevorzugtes Trägermaterial ist Mullit, Siliciumcarbid, $\alpha$-Aluminiumoxid und insbesondere Steatit. Man kann ebenfalls die sauerstoffhaltige Verbindung eines Zusatzelementes, z. B. Titan-(IV)-oxid oder Eisen-(III)-oxid, gleichzeitig als Träger für die katalytisch aktive Masse verwenden. Die Form und Größe der Trägerteilchen können in einem weiten Bereich beliebig gewählt werden, z. B. verwendet man tablettierte, körnige, stückige Trägerteilchen, Strangpreßlinge oder Pellets, vorteilhaft kugelförmige oder ringförmige Trägerteilchen, zweckmäßig von einem durchschnittlichen Durchmesser zwischen 2 und 10 Millimeter. Die katalytisch aktive Masse (Vanadium-V-Verbindungen, gegebenenfalls zusammen mit den Zusatzverbindungen) wird zweckmäßig auf dem Träger in einer Menge von 0,02 bis 0,5, vorzugsweise 0,15 bis 0,25 Gewichtsteile je Volumenteil Träger, aufgebracht.

In der Regel wird die aktive Masse (Vanadium-V-Verbindungen und gegebenenfalls Verbindungen der Zusatzelemente) in Form einer wäßrigen Suspension, vorteilhaft in einer von 7- bis 70-, vorzugsweise von 20- bis 40-gewichtsprozentigen Suspension zerstäubt. Als Zerteilungsorgan verwendet man zweckmäßig Zerstäuber und vorteilhaft Düsen, z. B. Injektormischer, Strahlmischer, Drallkammerdüsen, Exzenterdüsen, Bündeldüsen, Zentrifugal-Druckdüsen, Schlitzdüsen, Flachstrahldüsen, Hohldüsen, Spiraldüsen und insbesondere Zerstäubungsdüsen, Zweistoffdüsen, Rotationszerstäuber, Pralldüsen, Vollkegeldüsen, rotierende Zerstäuberscheiden mit Flügeln, rotierende Zerstäuberscheiben mit Düsen, Schlick-Düsen, Siccatom-Düsen, Hohlkegeldüse. Der Träger in Gestalt vorgenannter Trägerteilchen befindet sich während des Auftrags in ständiger Eigenbewegung. In der Regel erzielt man diese ständige Eigenbewegung durch sich drehende Behälter mit scheibenförmigen Boden, z. B. Drehtellern, Drehtrommeln, Beschichtungstellern, Imprägniertrommeln oder Dragiertrommeln, Wendelrührern oder Mischern, auf deren Boden sich die Trägerteilchen befinden. Der Winkel zur Horizontalen hängt vom verwendeten Apparat ab; bei Verwendung von Beschichtungs- oder Drehtellern stelle man den Behälterboden mit den Trägerteilchen mit einem Winkel zur Horizontalen ein, zweckmäßig 30 bis 70°, vorzugsweise 40 bis 50°, und erzielt somit neben der Fremdbewegung der Teilchen durch den sich drehenden Boden noch zusätzlich eine ständige erhebliche Eigenbewegung mittels der Schwerkraft. Kantige Teilchen werden sich daher bei der Bewegung ständig überschlagen, runde Teilchen, insbesondere Ringe oder Kugeln, eine rollende, gegebenenfalls kreiselnde Bewegung vollziehen. Da die Teilchen untereinander sich ständig durch Zusammenstöße an einer geradlinigen Bewegung hindern, resultiert aus allen diesen Bewegungsmomenten eine rollende Gesamtbewegung der Trägerteilchen, wie sie beispielsweise auch beim Beschichten von Schüttgut in Dragiertrommeln zu beobachten ist. Bevorzugt sind daher auch die leichter rollenden Teilchen wie Ringe und insbesondere Kugeln. Ein Unterschied zu Imprägnierungen und Dragierungen mit Lösungen wird bei dem erfindungsgemäßen Verfahren insbesondere auch darin gesehen, daß der Auftrag mit einem Gemisch von aktiver Masse und Wasser stattfindet, in dem die Masseteilchen im Wasser ganz oder

teilweise ungelöst sind und dementsprechend eine Suspension bilden. Die Suspension befindet sich bei der Zerstäubung vorteilhaft bei einer Temperatur von 10 bis 90° C, die Temperatur bei dem Auftrag der aktiven Masse, zweckmäßig gemessen als die Temperatur innerhalb der Schüttung, beträgt vorteilhaft von 20 bis 90° C, insbesondere 40 bis 70° C. Als Schüttung wird hier und im folgenden die Gesamtmasse der zu beschichteten Teilchen bzw. die Gesamtmasse der Schalenkatalysatoren, die Oberfläche der Gesamtmenge als Schüttungsoberfläche bezeichnet. Die zerstäubte Suspension wird auf die rollenden Trägerteilchen mit solcher Geschwindigkeit gesprüht, daß je Minute von 0,001 bis 0,02, vorzugsweise von 0,005 bis 0,015 Gewichtsteile Feststoff je Volumenteil Trägerkugeln aufgetragen werden. Durch die ständige rollende Bewegung wird gleichzeitig eine vergleichsweise gleichförmige Schicht von aktiver Masse aufgetragen. Aufgrund der Drehbewegung des Apparates werden die Trägerteilchen durch Wandreibkräfte und gegebenenfalls Einbauten emporgehoben und rollen infolge der Schwerkraft auf der Oberfläche der Schüttung ab bzw. bilden die Oberfläche der Schüttung. Der Apparat ist zweckmäßig während des Auftrags zu 20 bis 80 Volumenprozent mit Trägerteilchen gefüllt; die Drehung des Behälterbodens ist abhängig vom verwendeten Apparat und seiner Größe und beträgt im allgemeinen 2 bis 70 Umdrehungen pro Minute.

Anschließend werden die Teilchen durch direkte oder indirekte Beheizung getrocknet, zweckmäßig bis die Schüttungstemperatur 100 bis 110° C erreicht hat, vorteilhaft in demselben oder einem ähnlichen Drehbehälter, vorzugsweise Drehteller oder Drehtrommel. Nach dem Trocknen beträgt die Restmenge an Wasser in der aktiven Masse zweckmäßig 1 bis 10 Gewichtsprozent, bezogen auf die aktive Masse (gerechnet als Feststoff). Vorteilhaft trocknet man ganz oder teilweise während des Auftrags, z. B. durch Einblasen von Luft oder Inertgas, zweckmäßig bei einer Temperatur von 100 bis 300° C. Die Flammerhitzung oder Plasmaerhitzung der Schalenkatalysatoren wird mittels eines auf die Schüttungsoberfläche der Teilchen gerichteten Gas- oder Plasmabrenners durchgeführt, wobei sich die einzelnen Schalenkatalysatoren ebenfalls in Drehbehältern, zweckmäßig in denselben Drehbehältern, vorteilhaft Drehtellern, Imprägniertrommeln oder Dragiertrommeln, befinden. Vorteilhaft sind die vorgenannten Angaben bezüglich Winkel des Behälterbodens zur Horizontalen, Füllungsgrad, der Drehung des Bodens, Temperatur innerhalb des Schüttgutes unterhalb 650° C, vorteilhaft zwischen 650 und 300° C, zweckmäßig 650 und 500° C, vorzugsweise 650 und 600° C. Durch die rollende Bewegung der Schüttung trifft der Flamm- oder Plasmastrahl auf jeden Teil (Segment) der Oberfläche jedes einzelnen Schalenkatalysators nur kurzfristig, erhitzt dieses Segment auf eine Temperatur oberhalb 700° C, vorzugsweise zwischen 700 und 3000° C, zweckmäßig zwischen 700 und 1500° C, insbesondere zwischen 700 und 1000° C (Erhitzungstemperatur), hält ihn kurzfristig bei dieser Temperatur, dann werden die erhitzten Segmente wieder durch andere Segmente anderer Schalenkatalysatoren verdrängt, die nun ihrerseits auf die erfindungsgemäße Erhitzungstemperatur gebracht werden. Zweckmäßig werden die Einzelsegmente während 0,1 bis 1 Sekunden von der Temperatur innerhalb der Schüttung auf Erhitzungstemperatur gebracht und bei der Erhitzungstemperatur gehalten. Die so verdrängten Schalenkatalysatoren nehmen an der Oberfläche ihrer erhitzten Segmente rasch wieder die Temperatur innerhalb der Schüttung an. Jedes Segment der Schalenkatalysatoren kühlt sich zweckmäßig nach der Erhitzung während 0,1 bis 1 Sekunde auf die Abkühlungstemperatur, d. h. auf die Temperatur innerhalb der Schüttung, ab und verweilt bei dieser Temperatur bis zum nächsten Erhitzungsschritt. Die rollende Bewegung unterwirft somit alle Segmente aller Katalysatoren wiederholt der Behandlung mit dem Flammstrahl bzw. Plasmastrahl und anschließender rascher Abkühlung. Daher sind Formen der Schalenkatalysatoren, die eine gleichmäßige rollende Bewegung begünstigen, bevorzugt, d. h. runde Formen, insbesondere ringförmige, zylindrische und zweckmäßig kugelförmige oder annähernd kugelförmige (z. B. Pellets) Schalenkatalysatoren sind besonders vorteilhaft. Zweckmäßig verwendet man 10 bis 1000, vorzugsweise 20 bis 200 kg Schalenkatalysatoren sowohl bei dem Beschichtungsschritt wie auch bei der Erhitzung mittels Flamm- bzw. Plasmabehandlung und in Übereinstimmung damit eine Gesamtbehandlungszeit der Schalenkatalysatoren mit dem Gas- oder Plasmabrenner von 2 bis 60, vorzugsweise 10 bis 30 Minuten. Die Flamme des Gasbrenners kann z. B. mit Methan/Sauerstoff, Propan/Sauerstoff, Erdgas/Sauerstoff und vorteilhaft mit Acetylen/Sauerstoff oder Erdgas/Sauerstoff erzeugt werden. Statt Sauerstoff kann auch Luft verwendet werden. Vorteilhaft sind Durchmesser des Behälterbodens bei Verwendung von Drehtellern von 0,5 bis 5, insbesondere 0,5 bis 2 Meter, Abstände der Brenner von der Oberfläche zur Schüttung von 2 bis 70, vorzugsweise 30 bis 60 Zentimeter, Flammtemperaturen von 700 bis 2000° C.

Die so erhaltenen Schalenkatalysatoren werden vorteilhaft für die Herstellung von Anthrachinonen durch Oxidation von Indanen, zweckmäßig nach den in vorgenannten Patentschriften beschriebenen Verfahrensweisen, verwendet und besitzen hierbei eine hohe Aktivität und Selektivität. Vorteilhaft verwendet man insbesondere die vorgenannten bevorzugten Vanadium-Verbindungen und gewünschtenfalls bevorzugten weiteren Metallverbindungen und die vorgenannten Mengenverhältnisse.

Die Umsetzung läßt sich für den Fall der Verwendung von 1-Methyl-3-phenyl-indan durch die folgenden Formeln wiedergeben:

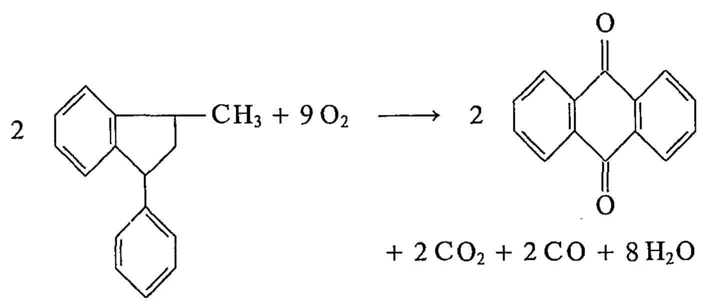

Die als Ausgangsstoffe I verwendeten Indane lassen sich durch Dimerisierung von substituierten oder unsubstituierten Styrolen, z. B. nach den in vorgenannten Veröffentlichungen oder in Rabjohn, Organic Syntheses, Collective Volume IV (John Wiley Inc., New York 1963), Seiten 665 ff beschriebenen Verfahren herstellen. Bevorzugte Indane I und dementsprechend bevorzugte Anthrachinone der Formel

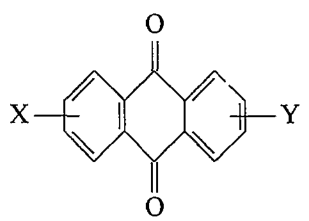

II

sind solche, in deren Formeln $R^1$, $R^2$, $R^3$ gleich oder verschieden sein können und jeweils einen Alkylrest mit 1 bis 4 Kohlenstoffatomen bedeuten, $R^1$ und/oder $R^3$ darüber hinaus auch jeweils ein Wasserstoffatom bezeichnen können, die Reste X und Y gleich oder verschieden sein können und jeweils ein Bromatom, ein Fluoratom oder insbesondere ein Wasserstoffatom oder ein Chloratom bezeichnen. Geeignete Indane I sind z. B. 1-Methyl-3-phenyl-indan, 1,3-Dimethyl-3-phenyl-indan, 1,1,3-Trimethyl-3-phenyl-indan, 1-Propyl-3-phenyl-indan, 1-Isobutyl-3-phenyl-indan; 3-o-Chlorphenyl-1-methyl-indan, 3-m-Chlorphenyl-1-methyl-indan, 3-p-Chlorphenyl-1-methyl-indan, 3-o-Bromphenyl-1-methyl-indan, 3-m-Bromphenyl-1-methyl-indan, 3-p-Bromphenyl-1-methyl-indan, 3-m-Chlorphenyl-1-methyl-4-chlor-indan, 3-m-Bromphenyl-1-methyl-4-brom-indan, 3-o-Chlorphenyl-1-methyl-7-chlor-indan, 3-o-Bromphenyl-1-methyl-7-brom-indan, 3-m-Chlorphenyl-1-methyl-6-chlor-indan, 3-m-Bromphenyl-1-methyl-6-brom-indan, 3-p-Chlorphenyl-1-methyl-5-chlor-indan, 3-p-Bromphenyl-1-methyl-5-brom-indan.

Die Oxidation wird in der Regel mit einem Sauerstoffüberschuß durchgeführt. Bevorzugt ist ein Verhältnis von 15 bis 400 Mol Sauerstoff je Mol Indan I über die stöchiometrische Menge. In der Regel verwendet man Sauerstoff in Gestalt von Luft, ebenfalls können beliebige Gemische von Sauerstoff und unter den Reaktionsbedingungen inerten Gasen wie Argon, Wasserdampf, Stickstoff und/oder Kohlendioxid oder Rauchgas zur Anwendung gelangen. Bevorzugt, insbesondere im Falle des 1-Methyl-3-phenyl-indans, beträgt die Beladung 5 bis 100, vorteilhaft 10 bis 60, insbesondere 25 bis 55 Gramm Ausgangsstoff I pro ein Normalkubikmeter Luft. Im Falle der halogenierten 1-Methyl-3-phenyl-indane kann die Beladung bevorzugt 5 bis 100, vorteilhaft 20 bis 70, insbesondere 30 bis 60 Gramm Ausgangsstoff I pro ein Normalkubikmeter Luft betragen. Im kontinuierlichen Betrieb werden zweckmäßig von 20 bis 2000, vorzugsweise 40 bis 1000, vorteilhaft 60 bis 600 Gramm Ausgangsstoff I pro Liter Katalysator und Stunde oxidiert. Die gleichen Mengen Ausgangsstoff I, bezogen auf den Katalysator, werden in der Regel auch im diskontinuierlichen Betrieb verwendet.

Die Oxidation wird in der Regel bei einer Temperatur zwischen 200 und 500°C, vorzugsweise zwischen 250 und 470°C, insbesondere zwischen 350 und 470°C, drucklos oder unter Druck, diskontinuierlich oder vorzugsweise kontinuierlich durchgeführt. Diese Temperatur wird in der Regel als Temperatur des Kühlmediums, z. B. eines Salpeterbades, gemessen (Rohrwandtemperatur). Man oxidiert z. B. den Ausgangsstoff I in der folgenden Weise: Das Ausgangsindan I wird verdampft und mit einem auf zweckmäßig 200 bis 400°C erhitzten Luftstrom vermischt. Es ist auch möglich, einen sauerstofffreien Teilstrom der Reaktionsabgase mit dem Dampf des Ausgangsstoffes zu sättigen und so die gewünschte Konzentration an Indan I im Reaktionsgemisch einzustellen. Das Gas-/Dampf-Gemisch wird dann in einem Reaktor bei der Reaktionstemperatur durch die Katalysatorschicht geleitet. Als Reaktoren kommen zweckmäßig mit einem Salzbad gekühlte Röhrenreaktoren, Wirbelschichtreaktoren mit eingebauten Kühlelementen oder Schichtreaktoren mit Zwischenkühlung in Frage. Aus dem Reaktionsgemisch wird nun in üblicher Weise der Endstoff abgetrennt, z. B. leitet man die den Reaktor verlassenden Gase durch einen oder mehrere Abscheider, um das Anthrachinon vom größten Teil der Nebenprodukte zu trennen. Gegebenenfalls ist eine Reinigung des Endstoffes möglich, indem man z. B. das Reaktionsgemisch in Wasser oder verdünnte Natronlauge leitet und aus dem dabei gebildeten festen Rückstand durch Sublimation den Endstoff isoliert oder den Endstoff in alkalischer

Natriumdithionitlösung löst, den unumgesetzten Ausgangsstoff filtriert, anschließend aus dem Filtrat den Endstoff durch Luftoxidation ausfällt und abtrennt. Vorzugsweise trennt man nach dem in der deutschen Patentschrift 2 232 453 beschriebenen Verfahren das Anthrachinon aus dem Gemisch seines Dampfes mit einem heißen Trägergas ab, indem man das auf eine Temperatur von mindestens 200°C erhitzte Gemisch von Anthrachinondampf und Trägergas mit einer wäßrigen, gegebenenfalls ammoniakalischen Suspension von Anthrachinon unterhalb der Siedetemperatur des Wassers beim herrschenden Druck behandelt.

Die erfindungsgemäß herstellbaren Anthrachinone sind wertvolle Ausgangsstoffe für die Herstellung von Farbstoffen und Schädlingsbekämpfungsmitteln. Bezüglich der Verwendung wird auf vorgenannte Veröffentlichungen und Ullmanns Encyklopädie der technischen Chemie, Band 3, Seiten 659 bis 732, verwiesen. Aus den Mono- und Dihalogenanthrachinonen können nach bekannten Verfahren durch Austausch der Halogenatome gegen Aminogruppen die entsprechenden Aminoanthrachinone erhalten werden, von denen sich, wie beispielsweise vom 1-Aminoanthrachinon, zahlreiche wertvolle Küpenfarbstoffe und lichtechte Lackpigmente ableiten.

Die in dem folgenden Beispiel aufgeführten Teile bedeuten Gewichtsteile. Sie verhalten sich zu den Volumenteilen wie Gramm zu Milliliter.

## Beispiel 1

a) Ein Gemisch aus 451,9 Teilen Vanadinpentoxid, 50,3 Teilen Ammoniumvanadat ($NH_4VO_3$), 8,0 Teilen Antimontrioxid und 0,98 Teilen Thalliumvanadat ($TlVO_3$) wird mit 1200 Teilen Wasser suspendiert. Die Suspension (20°C) wird mit einer Dosierpumpe zu einer Zweistoffdüse befördert, mit zugeführter Verdüsungsluft fein zerstäubt und auf die in einem rotierenden Beschichtungsteller (Winkel zur Horizontalen 47°, 15 Umdrehungen pro Minute) befindlichen Schüttung frei abrollender Trägerkugeln (Steatitkugeln von 6 Millimeter Durchmesser, Temperatur innerhalb der Schüttung 60°C) während 25 Minuten aufgesprüht. Der Drehteller ist zu 2/3 seines Volumens gefüllt. Die Geschwindigkeit des Auftrags beträgt 0,01 Teil Feststoff je Volumenteil Trägerkugeln und Minute. Das Wasser verdampft zum Teil während des Beschichtens. Anschließend wird noch 5 Minuten nachgetrocknet, bis die Temperatur innerhalb der Schüttung 105°C erreicht hat. Der fertige Katalysator enthält 0,15 Teile aktive Masse je Volumenteil Träger. Nun richtet man bei derselben Geschwindigkeit und Neigung des Drehtellers die Flamme eines Sauerstoff-Acetylen-Brenners während 5 Minuten mit 2700°C auf die Schüttung. Die Temperatur innerhalb der Schüttung beträgt 630°C, die Erhitzungstemperatur der Schüttungsoberfläche 750°C, die Aufheizzeit und Verweilzeit bei der Erhitzungstemperatur jedes kugelförmigen Schalenkatalysators beträgt pro Erhitzung zwischen 0,1 und 1 Sekunde, die Abkühlung pro Abkühlschritt und Schalenkatalysator beträgt 0,1 bis 1 Sekunde.

b) 63,72 Teile des so hergestellten Katalysators werden in einen Röhrenreaktor (21 mm innerer Durchmesser) eingefüllt. Nun wird ein Gemisch von 200 000 Volumenteilen Luft und 7,6 Teilen 1-Methyl-3-phenylindan stündlich durch den Katalysator geleitet. Die Rohrwandtemperatur beträgt 420°C. Das den Reaktor verlassende, gasförmige Reaktionsgemisch wird auf 50°C abgekühlt, wobei der Endstoff und das nicht umgesetzte 1-Methyl-3-phenylindan kondensieren. Der nicht kondensierte Anteil wird mit Wasser gewaschen. Nach dem Abdampfen des Waschwassers wird der verbliebene Rückstand mit dem Kondensat vereinigt.

Es werden nachstehende Ergebnisse erhalten:

Ausgangsstoff 1-Methyl-3-phenyl-indan: 38,10 Teile
Abgasmenge: 1 000 000 Volumenteile
Gehalt des Abgases an Kohlenmonoxid
und Kohlendioxid: 1,45 Volumen-%
Roher Endstoff: 36,28 Teile
Man erhält 74,1 Gewichtsprozent
Anthrachinon 26,88 Teile

(entspricht einer Anthrachinonausbeute, bezogen auf eingesetzten Ausgangsstoff, von 70,6% der Theorie).

## Vergleichsbeispiel 2
(analog zu DE-OS 2 547 655, Beispiel 1)

a) Herstellung des Katalysators
4,0 Teile Vanadinpentoxid werden unter Zugabe von 10,0 Teilen Oxalsäure in 200 Teilen Wasser gelöst, 96 Teile Titandioxid und 32 Teile Formamid zugesetzt und die gebildete Suspension auf Steatitkugeln von 5,4 mm Durchmesser (auf 300°C erhitzt) aufgetragen. Eine beschichtete Kugel

enthält 4 Teile aktive Masse (Vanadinpentoxid und Titandioxid) und 96 Teile Träger. Die aktive Masse setzt sich aus 96 Gew.-% Titandioxid und 4 Gew.-% Vanadinpentoxid zusammen.

b) Oxidation
67 Teile des gemäß a) hergestellten Katalysators werden in einen Röhrenreaktor (21 mm innerer Rohrdurchmesser) eingefüllt. Nun wird ein Gemisch von 200 000 Volumenteilen Luft und 7,4 Teilen 1-Methyl-3-phenylindan stündlich durch den Katalysator geleitet. Die Rohrwandtemperatur beträgt 371°C. Das den Reaktor verlassende, gasförmige Reaktionsgemisch wird auf 50°C abgekühlt, wobei der Endstoff und das nicht umgesetzte 1-Methyl-3-phenylindan kondensieren. Der nicht kondensierte Anteil wird mit Wasser gewaschen. Nach dem Abdampfen des Waschwassers wird der verbliebene Rückstand mit dem Kondensat vereinigt.

Es werden nachstehende Ergebnisse erhalten:

| | |
|---|---|
| Ausgangsstoff 1-Methyl-3-phenylindan: | 22,22 Teile |
| Abgasmenge: | 600 000 Vol.-Teile |
| Gehalt des Abgases an Kohlenmonoxid | |
| und -dioxid: | 1,40 Vol.-% |
| Roher Endstoff: | 19,51 Teile |

In dem rohen Endstoff werden durch UV-Absorption bestimmt:

54,5 Gew.-% Anthrachinon 10,63 Teile
17 Gew.-% Phthalsäureanhydrid 3,3 Teile
2,2 Gew.-% nicht umgesetzter Ausgangsstoff 0,43 Teile

(entspricht einem Umsatz von 98,1% und einer Anthrachinonausbeute, bezogen auf umgesetzten Ausgangsstoff von 48,8% der Theorie).

Vergleichsbeispiel 3
(analog zu DE-OS 2 547 624, Beispiel a)

c) Herstellung des Katalysators
600 Teile Steatit-Ringe mit einem äußeren Durchmesser von 8 mm, einer Länge von 6 mm und einer Wandstärke von 1,5 mm werden in einer Dragiertrommel auf 260°C erhitzt und mit einer Suspension, bestehend aus 400 g Anatas, 31 Teilen Vanadinpentoxid, 500 Teilen Wasser, 100 Teilen Formamid, 0,89 Teilen Rubidiumcarbonat und 10,85 Teilen Antimontrioxid besprüht, bis das Gewicht der aufgetragenen katalytisch aktiven Masse 10% vom Gesamtgewicht des Katalysators ausmacht.
Der Vanadinpentoxidgehalt des Trägerkatalysators beträgt 0,70 Gew. %.

d) Oxidation
42,6 Teile des gemäß Beispiel c) hergestellten Katalysators werden in einen Röhrenreaktor (21 mm innerer Rohrdurchmesser) eingefüllt. Nun wird ein Gemisch von 200 000 Volumenteilen Luft und 8,2 Teilen 1-Methyl-3-phenylindan stündlich durch den Katalysator geleitet. Die Rohrwandtemperatur beträgt 375°C. Das den Reaktor verlassende, gasförmige Reaktionsgemisch wird auf 50°C abgekühlt, wobei der Endstoff und das nicht umgesetzte 1-Methyl-3-phenylindan kondensieren. Der nicht kondensierte Anteil wird mit Wasser gewaschen. Nach dem Abdampfen des Waschwassers wird der verbliebene Rückstand mit dem Kondensat vereinigt.

Es werden nachstehende Ergebnisse erhalten:

| | |
|---|---|
| Ausgangsstoff 1-Methyl-3-phenylindan | 40,81 Teile |
| Abgasmenge | 1 000 000 Vol.-Teile |
| Gehalt des Abgases an Kohlenmonoxid | |
| und -dioxid | 2,43 Vol.-% |
| Roher Endstoff | 23,67 Teile |

In dem rohen Endstoff werden bestimmt:

72,0 Gew.-% Anthrachinon 17,04 Teile

(entspricht einer Anthrachinonausbeute, bezogen auf eingesetzten Ausgangsstoff von 41,8% der Theorie).

**Patentansprüche**

1. Verfahren zur Herstellung von Schalenkatalysatoren, bestehend aus einem nicht oder wenig porösen Träger und einer darauf angebrachten Schicht aus katalytisch aktiver, Vanadium-V-Verbindungen enthaltender Masse, wobei man Vanadium-V-Verbindungen im Gemisch mit Wasser zerstäubt und das so zerstäubte Gut auf sich in ständiger Eigenbewegung befindende Trägerteilchen aufbringt, dadurch gekennzeichnet, daß man das Gut bei einer Temperatur von 20 bis 90°C und mit einer Auftragsgeschwindigkeit von 0,001 bis 0,02 Gewichtsteilen Feststoff je Volumenteil Trägerkugel mal Minute als Schale aufträgt, dann die so erhaltenen Schalenkatalysatoren trocknet und mit einer Gesamtbehandlungszeit der Schalenkatalysatoren von 2 bis 60 Minuten mittels einer Flamm- oder Plasmaerhitzung der sich in ständiger Eigenbewegung befindenden Schalenkatalysatoren wiederholt alle Teile der Katalysatoroberfläche kurzfristig auf eine Temperatur oberhalb 700°C erhitzt und während 0,1 bis 1 Sekunde auf eine Temperatur unterhalb 650°C abkühlt.

2. Verwendung der Schalenkatalysatoren nach Anspruch 1 zur Herstellung von Anthrachinonen durch Oxidation von Indanen der Formel

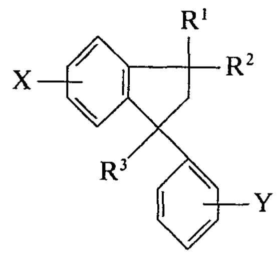

in der $R^1$, $R^2$ und $R^3$ gleich oder verschieden sein können und jeweils einen Alkylrest bedeuten, $R^1$ und/oder $R^3$ darüber hinaus auch jeweils ein Wasserstoffatom bezeichnen können, die Reste X und Y gleich oder verschieden sein können und jeweils ein Halogenatom oder ein Wasserstoffatom bedeuten, mit Sauerstoff in der Gasphase in Gegenwart von Vanadium-V-Verbindungen und gewünschtenfalls von einer oder mehreren Verbindungen weiterer Metalle.

**Claims**

1. A process for the production of a coated catalyst, comprising a non-porous or slightly porous carrier and a layer of catalytically active material, containing vanadium-V compounds, applied thereto, a mixture of water and a vanadium-V compound being atomized, and the atomized material being applied to carrier particles which are in constant motion, characterized in that the material is applied as a coating at from 20 to 90°C and with a rate of application of from 0.001 to 0.02 part by weight of solids per part by volume of carrier beads per minute, the coated catalysts thus obtained are then dried and all parts of the catalyst surface are repeatedly heated briefly to above 700°C by flame heating or plasma heating of the coated catalyst particles which are in constant motion, the total treatment time of the coated catalysts being from 2 to 60 minutes, and cooled to below 650°C in the course of 0.1 to 1 second.

2. Use of a coated catalyst as claimed in claim 1 for the preparation of an anthraquinone by oxidation of an indan of the formula

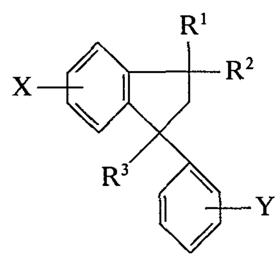

where $R^1$, $R^2$ and $R^3$ may be identical or different and each is alkyl, $R^1$ and/or $R^3$ may in addition also each be hydrogen and X and Y may be identical or different and each is halogen or hydrogen, by means of oxygen in the gas phase in the presence of an vanadium-V compound and, if desired, one or more compounds of other metals.

**Revendications**

1. Procédé de préparation de catalyseurs à particules enrobées, composés d'un support non poreux ou peu poreux portant une couche d'une matière à activité catalytique, comprenant des composés du vanadium pentavalent, dans lequel un mélange de composés du vanadium pentavalent et d'eau est pulvérisé sur les particules du support maintenues en mouvement permanent, caractérisé en ce que l'on réalise l'enrobage des particules du support à une température comprise entre 20 et 90° C et avec une vitesse de dépôt de 0,001 à 0,02 partie en poids de matière solide par partie en volume de particules et par minute, on sèche ensuite les particules enrobées obtenues, puis on soumet le catalyseur à particules enrobées, maintenues en mouvement permanent, avec une durée totale des traitements de 2 à 60 minutes, à des chauffages répétés de courte durée, à l'aide de flammes ou d'un plasma, portant toutes les parties des surfaces des particules de catalyseur à une température supérieure à 700° C, chaque chauffage étant suivi d'un refroidissement rapide, en 0,1 à 1 seconde, à une température inférieure à 650° C.

2. Utilisation des catalyseurs à particules enrobées suivant la revendication 1 pour la préparation d'anthraquinones par oxydation d'indanes de la formule

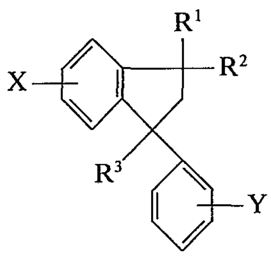

dans laquelle $R^1$, $R^2$ et $R^3$, qui peuvent être identiques ou différents, désignent chacun un radical alcoyle, $R^1$ et (ou) $R^3$ pouvant en outre représenter chacun un atome d'hydrogène, et X et Y, qui peuvent être identiques ou différents, représentent chacun un atome d'halogène ou un atome d'hydrogène, en phase gazeuse par l'oxygène en présence de composés du vanadium pentavalent et, le cas échéant, d'un ou de plusieurs composés d'autres métaux.